# EUROPEAN PATENT APPLICATION

(11) **EP 4 108 245 A1**
(43) Date of publication of application: **28.12.2022**
(21) Application number: 22748920.0
(22) Date of filing: 24.01.2022
(51) Int. Cl.: A61K 31/7072, A61K 31/708, A61K 31/7068, A61K 31/7076, A61P 11/02, A61P 37/08, A23L 33/13, A23L 2/52, A23C 9/16, A23C 9/152, A21D 13/06

(54) **NEW USE OF NUCLEOTIDE**

(30) Priority: 02.02.2021 CN 202110145510
(71) Applicant: Chen, Yusong, Liaoning 116635 (CN)
(72) Inventor: LI, Yong, Dalian, Liaoning 116635 (CN); LIU, Xinran, Dalian, Liaoning 116635 (CN); XU, Meihong, Dalian, Liaoning 116635 (CN); CHEN, Yusong, Dalian, Liaoning 116635 (CN); ZENG, Zheng, Dalian, Liaoning 116635 (CN)
(74) Representative: Proi World Intellectual Property GmbH
(86) International application number: PCT/CN2022/073440
(87) International publication number: WO 2022/166641

(57) **Abstract**

The present invention discloses new applications of a nucleotide in preparation of drugs for preventing and treating allergic rhinitis and other diseases or in functional foods for relieving allergic rhinitis symptoms. The nucleotide is a mixture of five nucleotides, i.e. CMP, AMP, UMP, GMP and IMP, with a small molecular weight, rapid absorption into the human body and high bioavailability. The present invention discovers that the intake of nucleotide can play a role of treating allergic diseases by alleviating allergic rhinitis symptoms, relieving allergy-induced splenomegaly, significantly reducing histamine levels in serum and nasal lavage fluid, and effectively regulating inflammatory factors. The nucleotide can be applied in preparation of drugs for preventing and treating allergic rhinitis and other diseases or in functional foods for relieving allergic rhinitis symptoms, and provides a new pathway for preventing and treating allergic rhinitis and other diseases by means of dietary intervention.

## Description

### TECHNICAL FIELD

The present invention relates to the technical fields of drug development and functional foods, and in particular to new applications of nucleotides.

### BACKGROUND

Allergic diseases refer to a series of diseases with tissue or organ dysfunction or damage caused by a large number of antibodies produced after exposure of the body to one or more sensitizing substances (allergens), mainly including allergic rhinitis and asthma, atopic dermatitis, allergic gastrointestinal diseases and food allergies. Allergic diseases have become a universal public health problem with an increasing incidence year by year, imposing a huge medical burden to patients. At present, the most common method for treating allergic diseases is chemotherapy, which relieves conditions to some extent, but cannot change the progression of allergic diseases and has no long-term sustained effects after drug withdrawal, with a high risk of relapse. In addition, as chemotherapy may have an adverse impact on the body when used for a long term, patients cannot be fundamentally cured. Therefore, it is quite necessary to explore an effective method that can effectively relieve or treat allergic diseases.

Studies have found that the intake of nucleotides can play a role of preventing and treating allergic rhinitis by alleviating allergic rhinitis symptoms in mice, relieving allergy-induced splenomegaly, significantly reducing histamine levels in serum and nasal lavage fluid, and effectively regulating inflammatory factors. If applied to the health industry, this technology can fully satisfy the health care and medical needs of patients with allergic diseases.

### SUMMARY

The present invention is intended to provide applications of a nucleotide preparation with a relatively low molecular weight and fast absorption in preparation of drugs for preventing and treating allergic rhinitis and other diseases or in functional foods for relieving allergic rhinitis symptoms, so as to provide a new pathway for preventing and treating allergic rhinitis and other diseases by means of dietary supplements. Moreover, a stable, effective and repeatable experimental animal model method is established.

In order to realize the aforesaid purpose, the present invention adopts the following technical solution:
In one aspect, the present invention provides an application of a nucleotide in preparation of drugs for preventing and treating allergic rhinitis.

In the other aspect, the present invention provides an application of a nucleotide in functional foods for relieving allergic rhinitis symptoms.

In the technical solution, further, the nucleotide is a nucleotide mixture of four or five 5'-mononucleotides or sodium salts thereof, and a total amount of various nucleotides calculated according to CMP, AMP, UMP, GMP and IMP molecules is 100%, wherein effective content percentages (by weight) of various nucleotides calculated according to CMP, AMP, UMP, GMP and IMP molecules are respectively as follows: 23-78% of CMP, 6-44% of AMP, 7-40% of UMP, 7-51% of GMP and 0-2.5% of IMP

In the technical solution, further, the effective content percentages of various nucleotides calculated according to CMP, AMP, UMP, GMP and IMP molecules are respectively as follows: 42-43% of CMP, 16-17% of AMP, 21-22% of UMP, 18% of GMP and 0-2.5% of IMP

In the technical solution, further, the effective content percentages of various nucleotides calculated according to CMP, AMP, UMP, GMP and IMP molecules are respectively as follows: 43% of CMP, 17% of AMP, 21% of UMP, 18% of GMP and 1% of IMP

In the technical solution, further, the drugs are in the forms of powders, tablets, soft capsules, hard capsules, or oral liquids.

In the technical solution, further, the functional foods are in the forms of powders or liquid beverages, and preferably in the forms of milk powders, dairy products or bakery products.

In the technical solution, further, the nucleotide can significantly reduce histamine (HIS) contents in serum and nasal lavage fluid of patients with allergic rhinitis symptoms, thereby greatly decreasing IgG, IgE, IL-4 and IL-12 in serum while increasing IL-10. The experimental animal model used is a model of allergic rhinitis in BALB/c mice induced by a specific amount of ovalbumin (OVA) combined with a specific amount of aluminum hydroxide gel immunoadjuvant.

The present invention discovers the nucleotide (an existing substance) having the functions of preventing and treating allergic rhinitis and other diseases, and their application in preparation of drugs for preventing and treating allergic rhinitis and other diseases or in functional foods for relieving allergic rhinitis symptoms. Animal experiments have confirmed that the intake of the nucleotide can play a role of preventing and treating allergic diseases by relieving allergy-induced splenomegaly, significantly reducing histamine levels in serum and nasal lavage fluid, and effectively regulating inflammatory factors, which shows significant effects of the nucleotide on prevention and treatment of allergic rhinitis and other diseases.

### BRIEF DESCRIPTION OF THE DRAWINGS

FIG. 1 shows effects of the nucleotide on HIS contents in mouse serum and nasal lavage fluid.
FIG. 2 shows effects of the nucleotide on IgE contents in mouse serum.
FIG. 3 shows effects of the nucleotide on IgG contents in mouse serum.

### DETAILED DESCRIPTION OF THE EMBODIMENTS

The present invention will be further described below in conjunction with specific embodiments, but those specific embodiments will not constitute a limitation to the present invention in any way.

### Example 1

1. The nucleotide of this example is a mixture of five 5'-mononucleotides or sodium salts thereof at the following ratio of content percentages (by weight): 43% of CMP, 17% of AMP, 21% of UMP, 18% of GMP and 1% of IMP.
2. The specific preparation method is as follows:
   (1) The five 5'-mononucleotides or sodium salts thereof were detected respectively, and reserved for later use after passing the detection.
   (2) The qualified five 5'-mononucleotides or sodium salts thereof were passed through a 60-mesh sieve, and reserved for later use.
   (3) The required amounts of mononucleotide samples were calculated and weighed according to the ratio, fully added and mixed for no less than 40 min. The obtained sample was stored at room temperature.

### Example 2

The nucleotide of this example is a mixture of five 5'-mononucleotides or sodium salts thereof at the following ratio of content percentages (by weight): 78% of CMP, 6% of AMP, 8% of UMP, 7% of GMP and 1% of IMP. The preparation method is the same as that in example 1.

### Example 3

The nucleotide of this example is a mixture of five 5'-mononucleotides or sodium salts thereof at the following ratio of content percentages (by weight): 23% of CMP, 44% of AMP, 25% of UMP, 7% of GMP and 1% of IMP. The preparation method is the same as that in example 1.

### Example 4

The nucleotide of the present invention is a mixture of five 5'-mononucleotides or sodium salts thereof at the following ratio of content percentages (by weight): 23% of CMP, 17% of AMP, 40% of UMP, 19% of GMP and 1% of IMP. The preparation method is the same as that in example 1.

### Example 5

The nucleotide of the present invention is a mixture of five 5'-mononucleotides or sodium salts thereof at the following ratio of content percentages (by weight): 24% of CMP, 17% of AMP, 7% of UMP, 51% of GMP and 1% of IMP. The preparation method is the same as that in example 1.

### Experimental Verification

### I. Materials and methods

1. Samples: nucleotide samples obtained in examples 1-5.
2. Experimental animals: 6-8-week-old BALB/c healthy female mice (SPF-grade, experimental animal use license No.: SYXK (Beijing) 2011-0039; experimental animal production license No.: SCXK (Beijing) 2011-0012) provided by the Experimental Animal Center of School of Medicine, Peking University. A total of 50 mice with a weight of 18-22 g were housed in separate cages (5 mice per cage) in the Experimental Animal Department of School of Medicine, Peking University. Food and water were supplied freely. The animal room had a temperature of 25ºC±1°C and a relative humidity of 50%-60%, and indoor lighting was controlled at a 12h/12h light-dark circadian rhythm.
3. Establishment of allergic rhinitis animal model: The model is a model of allergic rhinitis in mice induced by ovalbumin combined with 4% aluminum hydroxide gel immunoadjuvant. The specific process includes two parts: (1) Initial sensitization: On days 0, 7, 14 and 21, 200 µL of normal saline containing 25 µg of ovalbumin and 2 mg of aluminum hydroxide gel was intraperitoneally injected into mice for sensitization. (2) Rechallenge: On days 23, 25 and 27 after initial sensitization, 20 µL of normal saline containing 500 µg of ovalbumin was alternately dripped into bilateral nasal cavities of mice (10 µL on each side). The control group was given normal saline instead. The number of sneezing and nasal itching actions of mice was observed and recorded within 20 min after each challenge in nasal cavities. If the number of sneezing and nasal itching actions in the model group is significantly higher than that in the control group, the model will be deemed to be successfully established.
4. Groups and doses: Mice were randomly divided into 5 groups (i.e., blank control group, model control group, low-dose nucleotide group, medium-dose nucleotide group and high-dose nucleotide group) according to body weights, 10 mice per group. Mice in the blank control group were not subjected to the modeling process, while mice in the other groups were given respective test substances through intragastric administration at a fixed time every day after models were established. The low-dose nucleotide group was given the nucleotide according to 0.3 g/kg.bw, the medium-dose nucleotide group was given the nucleotide according to 0.6 g/kg.bw, and the high-dose nucleotide group was given the nucleotide according to 1.2 g/kg.bw. The model control group and blank control group were given the same amount of sterile distilled water. The intervention period was 14 days, during which food and water were supplied freely.
5. Main instruments and reagents: Bio-Rad microplate reader (USA); ovalbumin (USA), aluminum hydroxide gel adjuvant (China) and Elisa kit (China).
6. Experimental methods:
   6.1 Determination of body weight and organ coefficients: after 14 days of intragastric administration, mice were weighed, and sacrificed through cervical dislocation immediately after anesthetization and blood collection. The liver, kidney, spleen and thymus were collected, and weighed after fascias were removed and blood stains were removed with filter paper from the surfaces of the organs, and then the organ coefficient was calculated. The formula is that organ coefficient = (organ weight/body weight)^{∗}100.
   6.2 Determination of histamine contents in serum and nasal lavage fluid: after 14 days of intragastric administration, mice were anesthetized, then blood was collected into a 1.5 mL centrifuge tube, let stand and centrifuged, and serum was separated. The HIS content in serum was detected with the Elisa kit. Mice were sacrificed and then decapitated. Pre-cooled saline was drawn with a 1 mL syringe, carefully inserted into the nasopharynx from the trachea, and slowly pushed out. The nasal lavage fluid was received with a centrifuge tube at the nostrils and centrifuged, and the lavage fluid supernatant was separated. The HIS content in the nasal lavage fluid was detected with the Elisa kit.
   6.3 Determination of immunoprotein in serum: after 14 days of intragastric administration, mice were anesthetized, then blood was collected into a centrifuge tube, let stand and centrifuged, and serum was separated. IgG and IgE contents in serum were detected with the Elisa kit.
   6.4 Determination of inflammatory factors in serum: after 14 days of intragastric administration, mice were anesthetized, then blood was collected into a centrifuge tube, let stand and centrifuged, and serum was separated. IL-4, IL-10 and IL-12 contents in serum were detected with the Elisa kit.
7. Statistical method: All results are expressed as mean ± standard deviation. For the statistical test, one-way analysis of variance is conducted by SPSS 23.0 software. If p<0.05, the difference will be considered to be statistically significant.

### II. Experimental results of example 1

### 1. Effects of the nucleotide on body weight and organ coefficient of mice

It can be seen from the results in Table 1 that there are no statistically significant differences (p>0.05) in the body weight, kidney coefficient and thymus coefficient of mice in all groups. The liver coefficient of mice in the allergic model control group is significantly higher than that in the blank control group, while the liver coefficient of mice in three groups subjected to nucleotide intervention is significantly lower than that in the model control group and close to the level in the blank control group, indicating that nucleotide intervention can improve some liver damage symptoms that may occur in allergic mice.

The spleen coefficient of mice in the allergic model control group is significantly higher than that in the blank control group, while the spleen coefficient of mice in the low-dose nucleotide group subjected to intervention is significantly lower than that in the model control group, indicating that nucleotide intervention can alleviate allergy-induced splenomegaly, and has certain significance for the treatment of allergies.

**Table 1 Effects of the nucleotide on body weight and important organ coefficients of mice in example 1 (x̅±s, n=10)**

| Group | Body weight (g) | Liver (g/100 g) | Kidney (g/100 g) | Spleen (g/100 g) | Thymus (g/100 g) |
|---|---|---|---|---|---|
| Blank control group | 20.97±0.75 | 4.51±0.43^{b} | 1.21±0.14 | 0.41±0.04^{b} | 0.19±0.05 |
| Model control group | 20.64±0.97 | 5.24±0.43^{a} | 1.26±0.16 | 0.56±0.09^{a} | 0.23±0.07 |
| Low-dose nucleotide group | 20.33±0.72 | 4.60±0.52^{b} | 1.15±0.09 | 0.49±0.07^{ab} | 0.19±0.04 |
| Medium-dose nucleotide group | 20.25±0.68 | 4.65±0.27^{b} | 1.13±0.18 | 0.56±0.08^{a} | 0.20±0.03 |
| High-dose nucleotide group | 20.36±0.63 | 4.79±0.24^{b} | 1.22±0.07 | 0.55±0.05^{a} | 0.21±0.07 |

| | | | | | |
|---|---|---|---|---|---|
| Note: ^{a} statistically different from the blank control group, and ^{b} statistically different from the model control group. | | | | | |

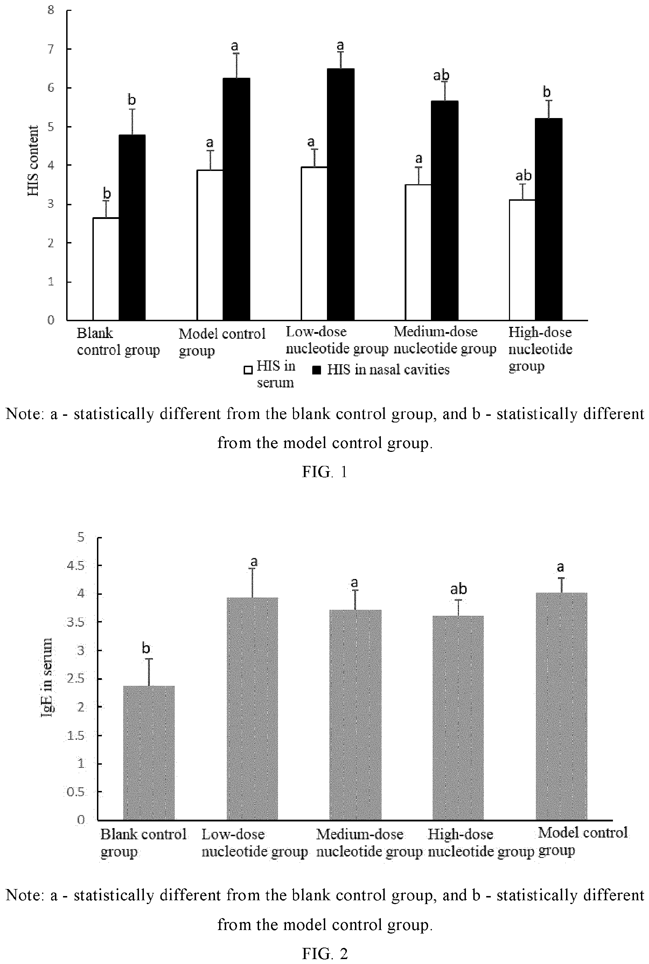

It can be seen from FIG. 1 that HIS contents in serum and nasal lavage fluid of mice in the model control group is significantly higher than that in the blank control group, indicating that the model is established very successfully and mice have obvious local and systemic allergic reactions. However, mice in the high-dose nucleotide group subjected to intervention exhibit a significant decrease in the HIS content in serum; on the other hand, the HIS contents in nasal lavage fluid of mice in medium-dose and high-dose nucleotide groups are also significantly decreased. This indicates that the intake of the nucleotide has certain effects on the treatment of allergies.

It can be seen from FIG. 2 that the IgE content in serum of mice in the high-dose nucleotide group is significantly lower than that in the model control group, indicating that nucleotide intervention has significant effects on the treatment of allergies.

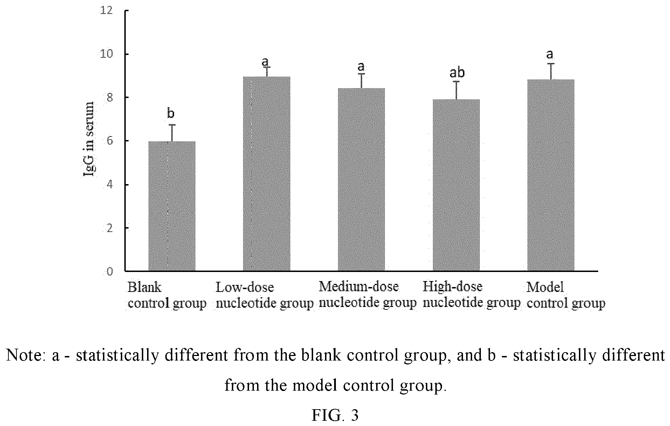

It can be seen from FIG. 3 that the IgG content in serum of mice in the high-dose nucleotide group is significantly lower than that in the model control group, indicating nucleotide intervention has significant effects on the treatment of allergies.

It can be seen from Table 2 that IL-4 and IL-12 levels in serum of mice in the high-dose nucleotide group is significantly lower than those in the allergic model control group.

The IL-10 level in serum of mice in the model control group is significantly lower than that in the blank control group, while the IL-10 level in serum of mice in the high-dose nucleotide group subjected to intervention is significantly higher than that in the model control group and close to that of healthy mice in the blank control group, indicating that nucleotide intervention has significant effects on the correction of disorders of inflammatory factors in serum of allergic mice.

**Table 2 Effects of the nucleotide on inflammatory factors in serum of mice in example 1 (x±s, n=10)**

| Group | IL-4 (pg/mL) | IL-10 (pg/mL) | IL-12 (pg/mL) |
|---|---|---|---|
| Blank control group | 69.44±6.42^{b} | 133.96±15.33^{b} | 35.13±2.92^{b} |
| Model control group | 89.19±6.90^{a} | 95.74±18.12^{a} | 46.24±4.66^{a} |
| Low-dose nucleotide group | 90.81±5.95^{a} | 98.02±12.66^{a} | 45.68±3.14^{a} |
| Medium-dose nucleotide group | 84.29±4.76^{a} | 98.39±8.74^{a} | 42.92±4.46^{a} |
| High-dose nucleotide group | 79.66±7.59^{ab} | 120.06±16.77^{b} | 38.03±4.05^{b} |

| | | | |
|---|---|---|---|
| Note: ^{a} statistically different from the blank control group, and ^{b} statistically different from the model control group. | | | |

### III. Experimental results of examples 2-5

It can be seen from Table 3 that IL-4 and IL-12 levels in serum of mice in the high-dose nucleotide groups in examples 2-5 are significantly lower than those in the allergic model control group.

The IL-10 level in serum of mice in the model control group is significantly lower than that in the blank control group, while the IL-10 level in serum of mice in the high-dose nucleotide groups subjected to intervention in examples 2-5 is significantly higher than that in the model control group and close to that of healthy mice in the blank control group, indicating that nucleotide intervention has significant effects on the correction of disorders of inflammatory factors in serum of allergic mice.

**Table 3 Effects of the nucleotide on inflammatory factors in serum of mice in examples 2-5 (x±s, n=10)**

| Group | IL-4 (pg/mL) | IL-10 (pg/mL) | IL-12 (pg/mL) |
|---|---|---|---|
| Blank control group | 72.52±5.62^{b} | 138.22±13.53^{b} | 37.32±3.12^{b} |
| Model control group | 92.58±6.30^{a} | 98.32±16.72^{a} | 49.44±5.16^{a} |
| High-dose nucleotide group in example 2 | 85.22±8.12^{ab} | 119.98±15.65^{b} | 43.62±4.31^{b} |
| High-dose nucleotide group in example 3 | 84.35±7.68^{ab} | 119.12±16.22^{b} | 42.18±4.02^{b} |
| High-dose nucleotide group in example 4 | 83.98±7.58^{ab} | 118.35±15.32^{b} | 43.98±4.56^{b} |
| High-dose nucleotide group in example 5 | 83.11±7.02^{ab} | 120.22±16.01^{b} | 41.87±4.15^{b} |

It can be seen from Table 4 that HIS contents in serum and nasal cavities of mice in the high-dose nucleotide groups in examples 2-5 are significantly lower than those in the allergic model control group.

**Table 4 Effects of the nucleotide on HIS contents in serum and nasal cavities of mice in examples 2-5 (x±s, n=10)**

| Group | HIS in serum (ng/mL) | HIS in nasal cavities (ng/mL) |
|---|---|---|
| Blank control group | 2.83±0.48^{b} | 4.32±1.12^{b} |
| Model control group | 3.56±0.5^{a} | 6.12±0.85^{a} |
| High-dose nucleotide group in example 2 | 3.32±0.51^{ab} | 5.63±0.95^{b} |
| High-dose nucleotide group in example 3 | 3.29±0.52^{ab} | 5.58±0.97^{b} |
| High-dose nucleotide group in example 4 | 3.26±0.53^{ab} | 5.50±0.89^{b} |
| High-dose nucleotide group in example 5 | 3.33±0.48^{ab} | 5.67±0.85^{b} |

### IV. Experimental conclusions

Taking the blank control group and model control group as control groups, this study explores the role of the nucleotide in prevention and treatment of allergic rhinitis and other diseases. The results of animal experiments show that the nucleotide can play a role of treating allergic diseases by relieving allergy-induced splenomegaly, significantly reducing histamine levels in serum and nasal lavage fluid, and effectively regulating inflammatory factors. This indicates that the nucleotide has significant effects on treatment of allergic rhinitis and other diseases, and a potential as a new drug for treating allergic diseases, and its concentration range is suitable for daily intake of 0.6-1.2 g/kg.bw.

Those skilled in the art may make many possible alterations and modifications to the technical solution of the present invention or changes the technical solution of the present invention into equivalent embodiments with equivalent variations using the technical contents disclosed above without departing from the scope of the technical solution of the present invention. Therefore, without departing from the contents of the technical solution of the present invention, all simple changes, equivalent variations and modifications made to the above-mentioned embodiments according to the technical essence of the present invention should still fall into the protection scope of the technical solution of the present invention.

## Claims

1. An application of a nucleotide in preparation of drugs for preventing and treating allergic rhinitis.

2. An application of a nucleotide in functional foods for relieving allergic rhinitis symptoms.

3. The application of claim 1 or 2, wherein the nucleotide is a nucleotide mixture of four or five 5'-mononucleotides or sodium salts thereof, and a total amount of various nucleotides calculated according to CMP, AMP, UMP, GMP and IMP molecules is 100%, wherein effective content percentages of various nucleotides calculated according to CMP, AMP, UMP, GMP and IMP molecules are respectively as follows: 23-78% of CMP, 6-44% of AMP, 7-40% of UMP, 7-51% of GMP and 0-2.5% of IMP

4. The application of claim 3, wherein the effective content percentages of various nucleotides calculated according to CMP, AMP, UMP, GMP and IMP molecules are respectively as follows: 42-43% of CMP, 16-17% of AMP, 21-22% of UMP, 18% of GMP and 0-2.5% of IMP

5. The application of claim 3, wherein the effective content percentages of various nucleotides calculated according to CMP, AMP, UMP, GMP and IMP molecules are respectively as follows: 43% of CMP, 17% of AMP, 21% of UMP, 18% of GMP and 1% of IMP

6. The application of claim 1, wherein the drugs are in the forms of powders, tablets, soft capsules, hard capsules, or oral liquids.

7. The application of claim 2, wherein the functional foods are in the forms of powders or liquid beverages, and preferably in the forms of milk powders, dairy products or bakery products.

8. The application of claim 1 or 2, wherein the nucleotides can significantly reduce histamine (HIS) contents in serum and nasal lavage fluid of patients with allergic rhinitis symptoms, and greatly decreases IgG, IgE, IL-4 and IL-12 in serum while increases IL-10.
